# EUROPEAN PATENT APPLICATION

(11) **EP 1 400 806 A1**
(43) Date of publication of application: **24.03.2004**
(21) Application number: 02021228.8
(22) Date of filing: 18.09.2002
(51) Int. Cl.: G01N 33/48

(54) **The use of molecular markers for the preclinical and clinical profiling of inhibitors of enzymes having histone deacetylase activity**

(71) Applicant: G2M Cancer Drugs AG, 60596 Frankfurt am Main (DE); Forschungszentrum Karlsruhe GmbH, 76344 Eggenstein-Leopoldshafen (DE)
(72) Inventor: Heinzel, Thorsten, Dr., 60529 Frankfurt am Main (DE); Krämer, Oliver H., 60318 Frankfurt am Main (DE); Göttlicher, Martin, Dr., 76297 Stutensee (DE); Zhu, Ping, 76351 Linkenheim (DE); Golebiewski, Martin, 76131 Karlsruhe (DE); Pelicci, Pier Giuseppe, Prof. Dr., 20090 Opera (Milan) (IT); Maurer, Alexander B., Dr., 61352 Bad Homburg (DE); Hentsch, Bernd, Dr., 60322 Frankfurt am Main (DE); Minucci, Saverio, Prof. Dr., 20090 Opera (Milan) (IT)
(74) Representative: Keller, Günter, Dr.

(57) **Abstract**

The present invention relates to the use of molecular markers and related signaling mechanisms for the preclinical and clinical profiling of inhibitors of enzymes having histone deacetylase activity. The invention also relates to the use of such markers as diagnostic and/or prognostic tools for the treatment of tumor patients with such inhibitors.

## Description

The present invention relates to the use of molecular markers and related signaling mechanisms for the preclinical and clinical profiling of inhibitors of enzymes having histone deacetylase activity. The invention also relates to the use of such markers as diagnostic and/or prognostic tools for the treatment of tumor patients with such inhibitors.

Local remodeling of chromatin is a key step in the transcriptional activation of genes. Dynamic changes in the nucleosomal packaging of DNA must occur to allow transcriptional proteins to contact with the DNA template. One of the most important mechanisms influencing chromatin remodeling and gene transcription are the posttranslational modification of histones and other cellular proteins by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). In the case of histone hyperacetylation, changes in electrostatic attraction for DNA and steric hindrance introduced by the hydrophobic acetyl group leads to destabilisation of the interaction of histones with DNA. As a result, acetylation of histones disrupts nucleosomes and allows the DNA to become accessible to the transcriptional machinery. Removal of the acetyl groups allows the histones to bind more tightly to DNA and to adjacent nucleosomes and thus to maintain a transcriptionally repressed chromatin structure. Acetylation is mediated by a series of enzymes with histone acetyltransferase (HAT) activity. Conversely, acetyl groups are removed by specific histone deacetylase (HDAC) enzymes. Disruption of these mechanisms gives rise to transcriptional misregulation and may lead to tumorigenic transformation.

Additionally, other molecules such as transcription factors alter their activity and stability depending on their acetylation status. E.g. PML-RAR, the fusion protein associated with acute promyelocytic leukemia (APL) inhibits p53 through mediating deacetylation and degradation of p53, thus allowing APL blasts to evade p53 dependent cancer surveillance pathways. Expression of PML-RAR in hematopoietic precursors results in repression of p53 mediated transcriptional activation, and protection from p53-dependent apoptosis triggered by genotoxic stresses (X-rays, oxidative stress). However, the function of p53 is reinstalled in the presence of HDAC inhibitors implicating active recruitment of HDAC to p53 by PML-RAR as the mechanism underlying p53 inhibition (Insinga et al. 2002, manuscript submitted). Therefore, factor acetylation plays a crucial role in the anti-tumor activity of HDAC inhibitors.

Nuclear hormone receptors are ligand-dependent transcription factors that control development and homeostasis through both positive and negative control of gene expression. Defects in these regulatory processes underlie the causes of many diseases and play an important role in the development of cancer. Many nuclear receptors, including T3R, RAR and PPAR, can interact with the corepressors N-CoR and SMRT in the absence of ligand and thereby inhibit transcription. Furthermore, N-CoR has also been reported to interact with antagonist-occupied progesterone and estrogen receptors. N-CoR and SMRT have been shown to exist in large protein complexes, which also contain mSin3 proteins and histone deacetylases (Pazin and Kadonaga, 1997; Cell 89, 325-8). Thus, the ligand-induced switch of nuclear receptors from repression to activation reflects the exchange of corepressor and coactivator complexes with antagonistic enzymatic activities.

The N-CoR corepressor complex not only mediates repression by nuclear receptors, but also interacts with additional transcription factors including Mad-1, BCL-6 and ETO. Many of these proteins play key roles in disorders of cell proliferation and differentiation (Pazin and Kadonaga, 1997, Cell 89, 325-8; Huynh and Bardwell, 1998, Oncogene 17, 2473-84; Wang, J. et al., 1998, Proc Natl Acad Sci U S A 95, 10860-5). T3R for example was originally identified on the basis of its homology with the viral oncogene v-erbA, which in contrast to the wild type receptor does not bind ligand and functions as a constitutive repressor of transcription. Furthermore, mutations in RARs have been associated with a number of human cancers, particularly acute promyelocytic leukemia (APL) and hepatocellular carcinoma. In APL patients RAR fusion proteins resulting from chromosomal translocations involve either the promyelocytic leukemia protein (PML) or the promyelocytic zinc finger protein (PLZF). Although both fusion proteins can interact with components of the corepressor complex, the addition of retinoic acid dismisses the corepressor complex from PML-RAR, whereas PLZF-RAR interacts constitutively. These findings provide an explanation why PML-RAR APL patients achieve complete remission following retinoic acid treatment whereas PLZF-RAR APL patients respond very poorly (Grignani et al., 1998, Nature 391, 815-8; Guidez et al., 1998, Blood 91, 2634-42; He et al., 1998, Nat Genet 18, 126-35; Lin et al., 1998, Nature 391, 811-4). Furthermore, a PML-RAR patient who had experienced multiple relapses after treatment with retinoic acid has recently been treated with the HDAC inhibitor phenylbutyrate, resulting in complete remission of the leukemia (Warrell et al., 1998, J. Natl. Cancer Inst. 90, 1621-1625).

By now, a clinical phase II trial with the closely related butyric acid derivative Pivanex (Titan Pharmaceuticals) as a monotherapy has been completed demonstrating activity in stage III/IV non-small cell lung cancer (Keer et al., 2002, ASCO, Abstract No. 1253). More HDAC inhibitors have been identified, with NVP-LAQ824 (Novartis) and SAHA (Aton Pharma Inc.) being members of the structural class of hydroxamic acids tested in phase I clinical trials (Marks et al., 2001, Nature Reviews Cancer 1, 194-202). Another class comprises cyclic tetrapeptides, such as depsipeptide (FR901228 - Fujisawa) used successfully in a phase II trial for the treatment of T-cell lymphomas (Piekarz et al., 2001, Blood 98, 2865-8). Furthermore, MS-27-275 (Mitsui Pharmaceuticals), a compound related to the class of benzamides, is now being tested in a phase I trial patients with hematological malignancies.

The recruitment of histone acetyltranferases (HATs) and histone deacetylases (HDACs) is considered as a key element in the dynamic regulation of many genes playing important roles in cellular proliferation and differentiation. Hyperacetylation of the N-terminal tails of histones H3 and H4 correlates with gene activation whereas deacetylation can mediate transcriptional repression. Consequently, many diseases have been linked to changes in gene expression caused by mutations affecting transcription factors. Aberrant repression by leukemia fusion proteins such as PML-RAR, PLZF-RAR, AML-ETO and Stat5-RAR serves as a prototypical example in this regard. In all of these cases, chromosomal translocations convert transcriptional activators into repressors, which constitutively repress target genes important for hematopoietic differentiation via recruitment of HDACs. It is plausible that similar events could also contribute to pathogenesis in many other types of cancer.

Mammalian histone deacetylases can be divided into three subclasses (Gray and Ekström, 2001). HDACs 1, 2, 3, and 8 which are homologues of the yeast RPD3 protein constitute class I. HDACs 4, 5, 6, 7, 9, and 10 are related to the yeast Hda 1 protein and form class II. Recently, several mammalian homologues of the yeast Sir2 protein have been identified forming a third class of deacetylases which are NAD dependent. All of these HDACs appear to exist in the cell as subunits of a plethora of multiprotein complexes. In particular, class I and II HDACs have been shown to interact with transcriptional corepressors mSin3, N-CoR and SMRT which serve as bridging factors required for the recruitment of HDACs to transcription factors.

### Molecular markers in cancer therapy

The discovery of new molecular markers for diagnosis and staging of human cancer is still an ongoing task and is essentiell for choosing the right therapeutic strategy. In the case of breast cancer, molecular markers such as the level of HER2/neu, p53, BCL-2 and estrogen/progesterone receptor expression have been clearly shown to correlate with disease status and progression. A newly approved kit measuring HER2 concentration in the serum of patients with metastatic breast cancer can now be used for followup and monitoring of these patients. Several large studies have shown that HER2 serum concentration is related to severity of disease, and - more importantly - in patients who respond to therapy, HER2 concentration decreases, irrespective of type of therapy. This example demonstrates the value of diagnostic and prognostic markers in cancer therapy.

### Medical need for new diagnostic and prognostic tools related to HDAC inhibitors.

The clinical benefits of HDAC inhibition and their implications for cancer therapy are currently being investigated in several locations. Although results from initial studies indicate that HDAC inhibitors may be benefical in the treatment of acute myeloid leukemia, T-cell lymphoma, and lung cancer it is highly likely that other cancer entities may be effectively treated. As yet, many of the HDAC inhibitors under investigation have often side effects demanding further development of new generation HDAC inhibitors. It is therefore essential to identify a characteristic profile for successful candidates as HDAC inhibitors. This may have dramatic consequences for saving cost and time in the development of new compounds. The second major task will then be to identify early on patients who will benefit from a therapy with these HDAC inhibitors and to monitor these patients during therapy. Both questions will be addressed in the present patent application.

The present invention aims at providing diagnostic and prognostic tools for the development and use of HDAC inhibitors. It has surprisingly been found that the level of certain proteins in cells treated with the HDAC inhibitor Valproic acid (VPA) is upregulated or downregulated in dependence of VPA treatment. These proteins or the RNA encoding them can be used as molecular markers. Therefore, one aspect of the present invention is the use of specific molecular markers for the profiling of HDAC inhibitors.

The invention relates to a method for the characterization of an HDAC inhibitor or a potential HDAC inhibitor comprising, determining in a sample the amount of a molecular marker, wherein the sample is derived from cells which have been treated with said HDAC inhibitor or potential HDAC inhibitor.

The term "molecular marker" as used herein designates a gene product, i.e. a protein or a ribonucleic acid including mRNA expressed from said gene, wherein the amount of said gene product in a cell is upregulated or downregulated by the HDAC inhibitor VPA in cell lines such as HEK 293T, F9, K562, HL60, and leukemic bone marrow. For example, VPA regulates expression of HDAC-2 and BCL-X_{L}, p21 (WAF), UBC8, RLIM, CASPASE 8, and TRAIL. Preferred markers according to the invention are HDAC-2 protein, Ubc8 RNA, UBC8 protein, RLIM protein, TRAIL RNA and TRAIL protein.

The "characterization of an HDAC inhibitor or potential HDAC inhibitor" encompasses the identification of patients and tumor entities that respond to a therapy with said HDAC inhibitors or potential HDAC inhibitors; monitoring efficacy of HDAC inhibitor treatment in patients; predicting therapeutic responses to HDAC inhibitors; profiling of HDAC inhibitors or potential HDAC inhibitors; and diagnosing diseases such as colon cancer.

The sample is a composition derived from cells which have been treated with an HDAC inhibitor or a potential HDAC inhibitor. The cells may be cell culture cells which have been contacted with said HDAC inhibitor or potential HDAC inhibitor. The inhibitor can be added to the growth medium of the cells. This embodiment is particularly suited for the profiling of HDAC inhibitors or potential HDAC inhibitors.

Another aspect of the present invention is the use of the above listed molecular markers to identify patients and tumor entities that respond to a therapy with these HDAC inhibitors. Furthermore, these markers can be used to monitor efficacy of HDAC inhibitor treatment in patients. Prognostic and diagnostic monitoring is suitable for diseases in which the induction of hyperacetylation of histones or other molecules has a beneficial effect resulting in differentiation and/or apoptosis of a patient's tumor cells and thus causing a clinical improvement of the patient's condition.

The cells may therefore also be derived from a tissue of an individual that was treated with an HDAC inhibitor. In this embodiment, the sample is preferably derived from tissue affected by a disorder. A tissue affected by a disorder is a tissue which differs from the corresponding tissue of a healthy individual. The difference may be a difference in morphology, histology, gene expression, response to treatment or protein composition etc. The tissue affected by the disorder may be tumor tissue in the case of cancer disease derived from but not restricted to bone marrow, colon, skin, breast, ovaries, prostate, kidney, bladder, esophagus, stomach, brain, lung, lymph nodes, and pancreas. Examples of such diseases include but are not limited to, skin cancer, melanoma, estrogen receptor-dependent and independent breast cancer, ovarian cancer, testosteron receptor-dependent and independent prostate cancer, renal cancer, colon and colorectal cancer, pancreatic cancer, bladder cancer, esophageal cancer, stomach cancer, genitourinary cancer, gastrointestinal cancer, uterine cancer, astrocytomas, gliomas, basal cancer and squameous cell carcinoma, sarcomas as Kaposi's sarcoma and osteosarcoma, head and neck cancer, small cell and non-small cell lung carcinoma, leukemia, lymphomas and other blood cell cancers.

The invention encompasses also the use of these markers for monitoring treatment of minimal residual tumor disease or tumor metastases.

Yet another aspect of the invention is the use of these markers in diseases that show aberrant recruitment of histone deacetylase activity such as thyroid resistance syndrome, or other conditions associated with abnormal gene expression, such as inflammatory disorders, diabetes, thalassemia, cirrhosis, protozoal infection, or the like and all types of autoimmune diseases, in particular rheumatoid arthritis, rheumatoid spondylitis, all forms of rheumatism, osteoarthritis, gouty arthritis, multiple sclerosis, insulin dependent diabetes mellitus and non-insulin dependent diabetes, asthma, rhinitis, uveithis, lupus erythematoidis, ulcerative colitis, Morbus Crohn, inflammatory bowel disease, as well as other chronic inflammations, chronic diarrhea.

Furthermore, the invention concerns the diagnostic and prognostic use of the above mentioned markers in other proliferative diseases such as psoriasis, fibrosis and other dermatological disorders. The terms "proliferative disease", and "cell proliferation", are used interchangeably herein and relate to an unwanted or uncontrolled cellular proliferation of excessive or abnormal cells which is undesired, such as, neoplastic or hyperplastic growth, whether *in vitro* or *in vivo.* Examples of proliferative conditions include, but are not limited to, pre-malignant and malignant cellular proliferation, including malignant neoplasms and tumors, cancers, leukemias, psoriasis, bone disease, fibroproliferative disorders (e.g. of connective tissues), and atherosclerosis. Any type of cell may be treated, including but not limited to, lung, colon, breast, ovarian, prostate, liver, pancreas, brain, and skin and any treatment of disorders involving T-cells such as aplastic anemia and DiGeorge syndrome, Graves' disease.

The cells are usually processed to be in a condition which is suitable for the method employed in determining the amount of molecular marker. Processing may include homogenization, extraction, fixation, washing and/or permeabilization. The way of processing largely depends on the method used for determination of the amount of molecular marker. The sample may be derived from a biopsy of the patient. The biopsy may be further treated to yield a sample which is in a condition suitable for the method used for determining the amount of molecular marker.

A variety of methods can be employed to determine the amount of molecular marker in the sample. The type of method to be used depends on the nature of the molecular marker. When the molecular marker is a protein the amount of the molecular marker is preferably determined by use of an antibody directed against the molecular marker.

As used herein, the term "antibody" designates an immunoglobulin or a derivative thereof having the same binding specificity. The antibody used according to the invention may be a monoclonal antibody or an antibody derived from or comprised in a polyclonal antiserum, monoclonal antibodies are preferred. The term "antibody", as used herein, further comprises derivatives such as Fab, F(ab')2, Fv, or scFv fragments: see, for example Harlow and Lane, "Antibodies, A Laboratory Manual" CSH Press 1988, Cold Spring Harbor N.Y. The antibody or the derivative thereof may be of natural origin or may be (semi)synthetically produced. Such synthetic products also comprises non-proteinaceous or semi-proteinaceous material that has the same or essentially the same binding specificity as the antibody of the invention. Such products may, for example be obtained by peptidomimetics. Methods of producing antibodies directed against the molecular marker proteins are known in the art (Harlow and Lane, "Antibodies, A Laboratory Manual" CSH Press 1988, Cold Spring Harbor N.Y.). The amino acid sequences of the molecular markers HDAC-2 protein, UBC8 protein, RLIM protein and TRAIL protein are described in Yang,W.M. et al., 1996, Proc. Natl. Acad. Sci. U.S.A. 93, 12845-12850; Kaiser, P. et al., 1994, J. Biol. Chem. 269, 8797-8802; Ostendorff, H.P et al., 2000, Genomics 69, 120-130; Boldin, M.P. et al., 1995, J. Biol. Chem. 270, 7795-7798; Mariani, S.M. et al., 1997, J Cell Biol 137, 221-229, respectively. The antibody may be used in methods which are known to those skilled in the art, e.g. Western Blotting, ELISA, immunohistochemistry and/or flow cytometry.

Western Blotting may be used which is generally known in the art. The cellular material or tissue may be homogenized and treated with denaturing and/or reducing agents to obtain the samples. The sample may be loaded on a polyacrylamide gel to separate the proteins followed by transfer to a membrane or directly be spotted on a solid phase. The antibody is then contacted with the sample. After one or more washing steps the bound antibody is detected using techniques which are known in the art. Gel electorphoresis of proteins and Western Blotting is described in Golemis, "Protein-Protein Interactions: A Laboratory Manual", CSH Press 2002, Cold Spring Harbor N.Y.

Immunohistochemistry may be used after fixation and permeabilisation of tissue material, e.g. slices of solid tumors. The antibody is then incubated with the sample, and following one or more washing steps the bound antibody is detected. The techniques are outlined in Harlow and Lane, "Antibodies, A Laboratory Manual" CSH Press 1988, Cold Spring Harbor N.Y..

In a preferred embodiment, the amount of molecular marker is determined by way of an ELISA. A variety of formats of the ELISA can be envisaged. In one format, the antibody is immobilized on a solid phase such as a microtiter plate, followed by blocking of unspecific binding sites and incubation with the sample. In another format, the sample is first contacted with the solid phase to immobilize the molecular marker proteins contained in the sample. After blocking and optionally washing, the antibody is contacted with the immobilized sample. ELISA techniques are described in Harlow and Lane, "Antibodies, A Laboratory Manual" CSH Press 1988, Cold Spring Harbor N.Y..

Most preferably, the amount of molecular marker is determined by flow cytometry. Cells, e.g. cell culture cells or blood cells or cells from bone marrow, are fixed and permeabilized to allow the antibody to reach the molecular marker proteins. After optional washing and blocking steps the antibody is contacted with the cells. Flow cytometry is then performed in accordance with procedures known in the art in order to determine cells having antibody bound to molecular marker proteins. Various flow cytometry methods are described in Robinson "Current Protocols in Cytometry" John Wiley & Sons Inc., New York.

When the molecular marker is a ribonucleic acid such as mRNA, nucleic acid technologies are usually employed to determine the amount of molecular marker in the sample. Preferably hybridization techniques and/or PCR techniques are employed. Northern blotting techniques may be used to determine the amount of RNA marker in the sample. In a preferred embodiment, RT-PCR is used. The man skilled in the art is able to design suitable primers and/or probes to be used in these methods on the basis of the nucleotide sequences of the respective markers. The cDNA sequences of Ubc8 and TRAIL are described in Boldin, M.P. et al., 1995, J. Biol. Chem. 270, 7795-7798; Mariani, S.M. et al., 1997, J Cell. Biol. 137, 221-229; Kaiser, P. et al., 1994, J. Biol. Chem. 269, 8797-8802, Ostendorff, H.P et al., 2000, Genomics 69, 120-130; respectively. Methods how to design primers and probes are described in Dieffenbach "PCR Primer: A Laboratory Manual" CSH Press 1995, Cold Spring Harbor N.Y..

In a further embodiment, the method comprises the step of selecting the inhibitor if it has the activity of modulating the expression of the molecular marker. "Modulating the expression of the molecular marker" as used herein designates the capability or activity of a compound to induce upon contact with a cell an increase or decrease in the amount of molecular marker in the cell. To determine whether an HDAC inhibitor or potential HDAC inhibitor has this acitivity, one usually determines the amount of molecular marker in a reference sample wherein the reference sample is derived from cells which have not been treated with said HDAC inhibitor or potential HDAC inhibitor. The determination of the amount of molecular marker in the sample and in the reference sample may be performed in parallel. In the case of cell culture cells, two cellular compositions are provided, one of which is treated with HDAC inhibitor or potential HDAC inhibitor, whereas the other is left untreated. Subsequently both compositions are further processed and the respective amounts of molecular marker are determined.

In the case of patients, the sample is derived from a patient which has been treated with HDAC inhibitor or potential HDAC inhibitor. The reference sample is derived from another patient suffering from the same disorder who has not been treated with said HDAC inhibitor or potential HDAC inhibitor or from a healthy individual. The tissue from which this reference sample is derived corresponds to the tissue from which the sample is derived. For example, if the sample is derived from tumor tissue from a breast cancer patient the reference sample is also derived from tumor tissue from a breast cancer patient or from breast tissue from a healthy individual. It may also be envisaged that the sample and the reference sample are derived from the same individual. In this case, the tissue, from which the reference sample is derived was obtained from the individual prior to or after treatment of the individual with an HDAC inhibitor. Preferably, the tissue was obtained prior to the treatment to exclude possible after-effects of the inhibitor treatment on the expression of the molecular marker after discontinuation of the treatment.

Another aspect of the invention is the use of a means for determining the amount of a molecular marker for profiling of HDAC inhibitors or potential HDAC inhibitors.

Methods for determining the amount of a molecular marker have been described supra. Thus, preferred means for determining the amount of a molecular marker are antibodies directed against molecular marker proteins, primers which can be used to amplify molecular marker RNA or cDNA derived therefrom, probes which are capable of specifically hybridizing to molecular marker RNA or cDNA derived therefrom. The most preferred means include antibodies directed against TRAIL protein, HDAC-2 protein, RLIM or UBC8 protein; primers capable of specifically amplifying TRAIL RNA, TRAIL cDNA, Ubc8 RNA or Ubc8 cDNA; and probes capable of specifically hybridizing to TRAIL RNA, TRAIL cDNA, Ubc8 RNA or Ubc8 cDNA under standard conditions. The primers and probes are usually oligonucleotides having a length of 10 to 50 nucleotides, preferably 15 to 30 nucleotides, more preferably 15 to 24 nucleotides. In one embodiment the oligonucleotides hybridize to the respective target sequence under conditions such as 0.1xSSC, 0.1% SDS and 65°C. The setting of appropriate conditions is described in Sambrook et al., "Molecular Cloning, A Laboratory Manual, 1989; or Hames and Higgins. "Nucleic acid hybridization, a practical approach", IRL press, 1985.

In yet another aspect the invention concerns the use of a means for determining the amount of a molecular marker for diagnosing a disease. Preferably, means for determining the amount of HDAC-2 protein are used for diagnosing colon cancer. In particular, the invention relates to the use of an antibody directed against HDAC-2 protein for diagnosing colon cancer.

The invention further relates to the use of a means for determining the amount of a molecular marker for determining whether a treatment of a disorder with an HDAC inhibitor is to be started/continued or not. This aspect includes the identification of patients and tumor entities that respond to a therapy with said HDAC inhibitor or potential HDAC inhibitor, monitoring efficacy of HDAC treatment in patients; predicting therapeutic response to HDAC inhibitors; and diagnosing diseases such as colon cancer.

The invention also concerns a diagnostic kit containing (i) means for determining the amount of a molecular marker; and (ii) an HDAC inhibitor. The preferred embodiments of the kit correspond to the preferred embodiments of the method or use of the invention as described supra.

Preferably, the method or use of the invention comprises only steps which are carried out in vitro. Therefore, according to this embodiment the step of obtaining the tissue material from the human or animal body is not encompassed by the present invention.

### Selective proteosomal degradation of HDAC-2 by HDAC inhibitors

Cancer therapy using HDAC inhibitors induces differentiation and/or apoptosis in tumor cells. This is achieved in a process involving hyperacetylation of HDAC-dependent substrates, such as histones and transcription factors (e.g. the tumor suppressor p53, which in turn regulate expression of genes that promote diffentiation or apoptosis. The present invention therefore also relates to the ability of certain HDAC inhibitors to induce the expression of the E2 ubiquitin conjugating enzyme Ubc8, and the concomitant specific proteasomal degradation of the iso-enzyme HDAC-2 via the E3 ubiquitin ligase RLIM, which adds a novel aspect to the activity of certain HDAC inhibitors, such as Valproic acid, acting synergistically with the enzymatic inhibition of histone deacetylases.

This invention also concerns the diagnostic use of Ubc8 and HDAC-2 expression in tumors from patients to predict therapeutic response to HDAC inhibitors, such as Valproic acid, which upregulate Ubc8 and downregulate HDAC-2 expression. It also includes the use of HDAC-2 expression in tumors to monitor efficacy of certain HDAC inhibitors, such as Valproic acid, in the course of therapy.

Furthermore, a particular aspect of the present invention relates to the profiling of HDAC inhibitors or compounds with assumed HDAC inhibitory activity for their ability to downregulate HDAC-2 and/or RLIM expression and upregulate Ubc8 expression. Methods to analyse expression of HDAC-2, RLIM and Ubc8 comprise the use of nucleic acid technology, preferably of hybridization or polymerase chain reaction for detection. Other types of nucleic acid technology, however, may be employed. In another embodiment of the invention the method comprises the use of specific antibodies against the above mentioned proteins for detection.

### Molecular markers for apoptotic pathways induced by HDAC inhibitors

Normal cells have an intrinsic mechanism of self destruction called programmed cell death or apoptosis. This balance is often disturbed in cancer cells inducing too much growth and too little death (Igney and Kramer, 2002, Nature Reviews Cancer 2:277-288). Understanding of these mechanisms at the molecular level does, ultimately, lead to new therapeutic approaches based on modulation of apoptosis sensitivity. Apoptosis can be induced by activation of, so called, death receptors that belong to the tumor-necrosis factor (TNF) receptor superfamily. Death receptors are activated by their natural ligands, the TNF family, including TNFalpha, CD95L and TRAIL. This leads to activation of a death-inducing signaling complex (DISC) containing caspase 8 which - in turn - induces apoptosis. Upregulation of any member of this pathway enhances the effect of pro-apoptotic signals.

The apoptotic process is tightly controlled by various regulatory proteins, such as the members of mitochondrial BCL2 family being among the most important. BCL2 family members can be divided into anti-apoptotic, such as BCL2, BCL-XL, BCL-w, and pro-apoptotic proteins, such as BAX, BAK, BID, BAD. Anti-apoptotic BCL2 family members seem to be involved in resistance of tumors to apoptosis. For example, BCL-XL can confer resistance to multiple apoptosis-inducing pathways in cancer cells and seems to be upregulated by a constitutively active epidermal growth factor receptor. High expression of BCL-XL is found in many human cancers and is often a negative prognostic factor. Accordingly, downregulation of BCL-XL expression in certain cancer cells either induces apoptosis directly or sensitizes cells to apoptotic stimuli.

This present invention relates to the ability of certain HDAC inhibitors, such as Valproic acid, to specifically induce the expression of the pro-apoptotic molecules TRAIL and Caspase 8 and to downregulate the expression of the anti-apoptotic molecule BCL-XL in cancer cells, which provides a deeper insight into the mechanism of induction of apoptosis and sensitization to apoptotic stimuli induced by these HDAC inhibitors. Additionally, the invention concerns the diagnostic use of TRAIL, Caspase 8 and BCL-XL expression in tumors from patients to predict therapeutic response to HDAC inhibitors, such as Valproic acid, that upregulate TRAIL and Caspase 8 expression and downregulate BCL-XL expression. It also includes the use of TRAIL, Caspase 8 and BCL-XL expression in tumors to monitor the efficacy of certain HDAC inhibitors, such as Valproic acid, in the course of therapy.

In yet another embodiment the invention relates to the profiling of HDAC inhibitors or compounds with assumed HDAC inhibitory activity for their ability to downregulate BCL-XL expression and upregulate TRAIL and Caspase 8 expression. Methods to analyse TRAIL, Caspase 8, and BCL-XL expression comprise the use of nucleic acid technology, preferably of hybridization or polymerase chain reaction for detection. Other types of nucleic acid technology, however, may be employed. In another embodiment the method comprises the use of specific antibodies against the above mentioned proteins for detection.
**Figure 1:** VPA enhances survival of acute promyelocytic leukemia mice (Figure 1A) and reduced splenomegaly in acute promyelocytic leukemia mice after treatment with VPA (Figure 1B)(Example 1).
**Figure 2:** Histone hyperacetylation in splenocytes in normal and leukemic mice after treatment with VPA (400 mg/kg) for three and six hours (Figure 2A and B) (Example 1).
**Figure 3:** VPA induces apoptosis in leukemic cells, but not in normal splenocytes in vivo (Figure 3A) and in vitro (Figure 3B) (Example 1).
**Figure 4:** VPA induces TRAIL expression in leukemic cells in vivo (Figure 4A), and in non-leukemic tumor cell lines in vitro (Figure 4B) (Example 1).
**Figure 5:** Inhibition of TRAIL and caspase 8 impair VPA-induced apoptosis of murine APL blasts (Example 1).
**Figure 6:** VPA but not TSA induces reduction of HDAC-2 protein levels (Example 2).
**Figure 7:** VPA does not change HDAC-2 mRNA levels (Example 2).
**Figure 8:** Induction of HDAC-2 protein degradation by VPA (Example 2).
**Figure 9:** Proteasome inhibitors but not protease inhibitors inhibit VPA induced HDAC-2 degradation (Example 2).
**Figure 10:** HDAC-2 is ubiquitinated in response to VPA treatment (Example 2).
**Figure 11:** VPA induces expression of the E2 ubiquitin conjugase Ubc8 (Example 3).
**Figure 12:** The HDAC inhibitor TSA, but not VPA induces proteosomal degradation of the E3 ubiquitinating enzyme RLIM (Example 3).
**Figure 13:** Identification of E3 ubiquitinating enzyme, RLIM to induce ubiquitination of HDAC's 1-3 (Example 3).
**Figure 14:** HDAC-2 is upregulated in human colon cancer tissues (Example 4).

The following examples further illustrate the invention:

### EXAMPLE 1

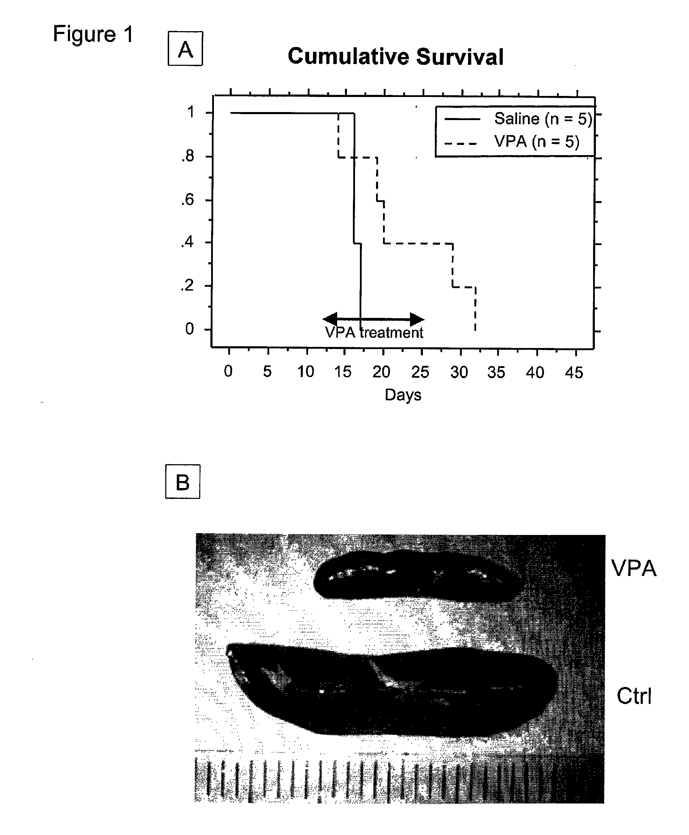

HDAC inhibitors induce TRAIL expression, Caspase 8 activation and apoptosis in cancer cells but not normal blood cells (Figure 1-5).

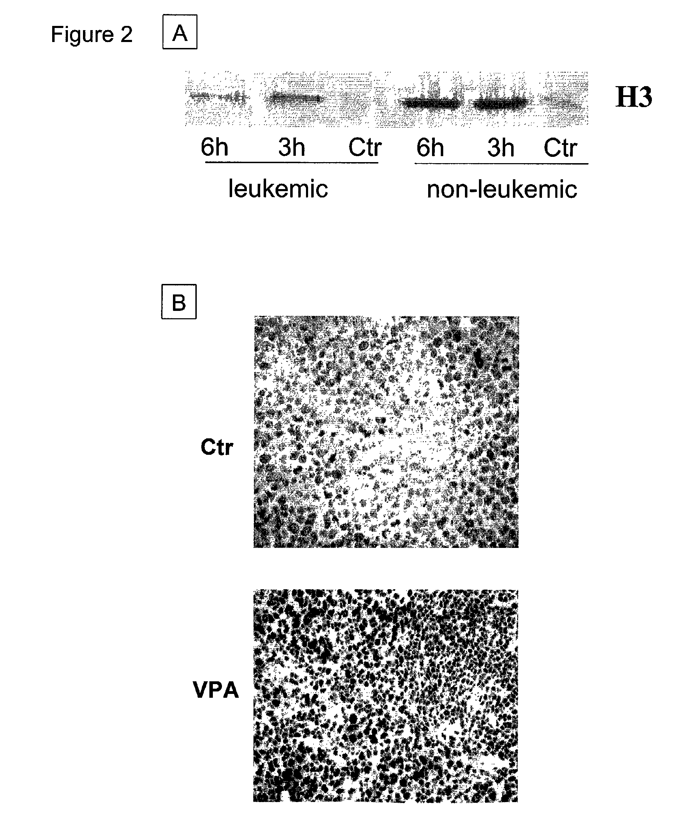

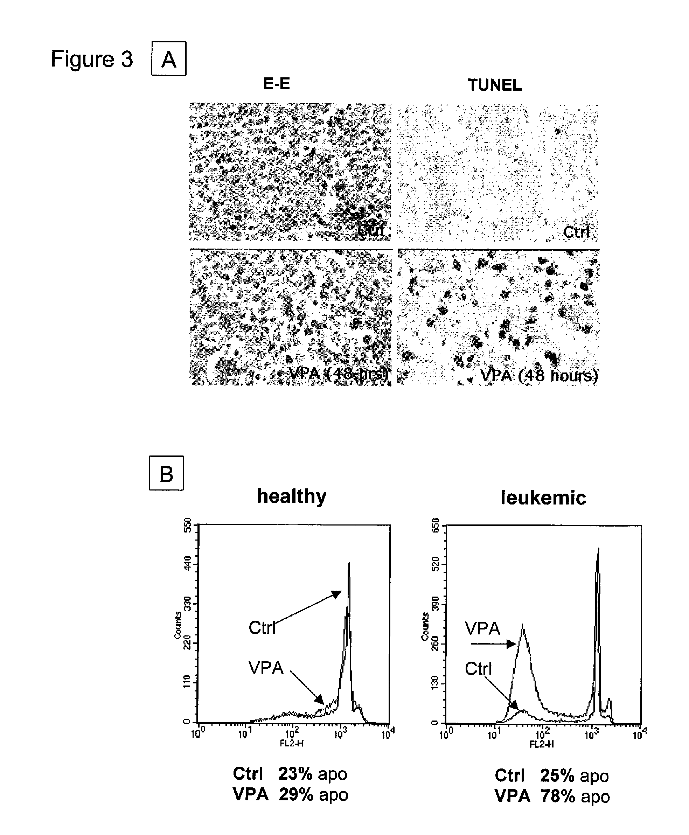

Although the discrete mechanism of action varies, cancer therapy still depends on an ability to engender apoptosis in cancer cells as a final common pathway. HDAC inhibitors have already been shown to induce apoptosis in certain cancer cells through down-regulation of the anti-apoptotic molecules, such as BCL-XL and BCL-2. In general, induction of apoptosis can be exploited therapeutically using HDAC inhibitors in cancer therapy. We present new evidence that HDAC inhitors (namely Valproic acid - VPA) can be used in cancer treatment using a mouse leukemia model of acute promyelocytic leukemia (APL; Figure 1). We demonstrate that HDAC inhibitors such as VPA, induce histone hyperacetylation (Figure 2) and apoptosis (Figure 3) in leukemic cells from mice with APL. According to the present invention, HDAC inhibitors (namely Valproic acid) induce apoptosis in leukemic cells cancer cells by upregulating TRAIL expression and activating caspase 8. This process is not seen in normal cells.

### METHODS

**Figure 1:** (A) Secondary acute promyelocytic leukemias were induced in mice. In brief, primary leukemias were induced in mice by injecting PML/RARα expressing bone marrow cells into the tail veins. Mice with overt leukemias were euthanized, and leukemic cells were harvested from the spleen. Leukemic cells were re-injected i.v. (1x10⁷ cells/mouse) in non-irradiated, syngeneic recipient mice. The secondary recipient mice developed overt leukemia in about 2-3 weeks. Leukemic mice or healthy mice (Ctrl) were then treated with placebo (lanes 1, 3), or VPA (400 mg/kg every 12 hours) for two consecutive weeks. (B) Leukemic mice were treated with placebo (lower, Ctrl) or VPA (400 mg/kg every twelwe hours, upper) for one week. At the end of the treatment, mice were sacrificed, and spleens were photographed.
**Figure 2:** (A) Secondary acute promyelocytic leukemias were induced in mice as described above. (A) Leukemic mice or healthy mice (Ctrl) were then treated with placebo or VPA (400 mg/kg) for three and six hours. Mice were then sacrificed, whole cell extracts were prepared from the spleen (with >70% leukemic infiltration), and analyzed by SDS-PAGE followed by Western blot analysis using commercially available antibodies against acetylated histone H3. (B) Immunohistochemistry analysis of VPA-treated leukemic mice using a monoclonal antibody against acetylated histones.
**Figure 3:** (A) Secondary acute promyelocytic leukemias were induced in mice as described above. Leukemic mice were then treated with placebo (Ctrl), or VPA (400 mg/kg every 12 hours). Mice were sacrificed 48 hours after beginning of treatments, and then leukemic spleens were fixed and either stained for histological examination (hematoxylin-eosyn, left panels), or analyzed for the presence of apoptotic cells (TUNEL assay, right panels). (B) Secondary leukemias were obtained as above. Mice were sacrificed when leukemic invasion of peripheral organs was >75% (spleen). Splenocytes from healthy mice (taken as control, left panel), or from leukemic mice (>75% blasts, right panel) were put in culture and then treated with VPA (1mM) for 48 hours. At the end of the treatment, apoptosis was measured by propidium iodide staining. The sub-G1 area indicates the hypodiploid DNA peak corresponding to cells with fragmented DNA undergoing apoptosis.

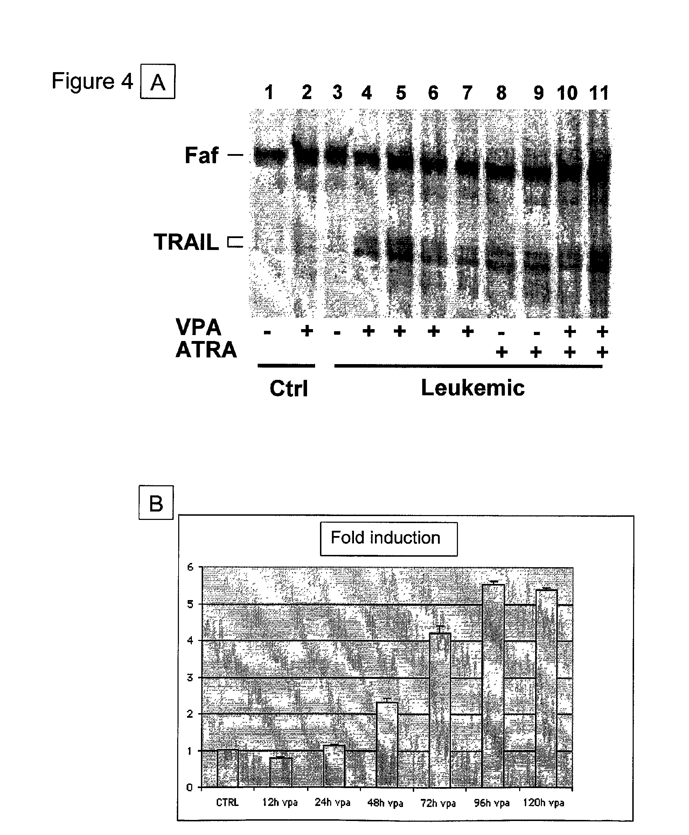

**Figure 4:** (A) Secondary acute promyelocytic leukemias were induced in mice as described above. Leukemic mice or healthy mice (Ctrl) were then treated with placebo (lanes 1, 3), VPA (400 mg/kg every 12 hours), all-trans retinoic acid (a 21-day-release pellet containing 5 mg RA or placebo: lanes 8-9), and VPA plus retinoic acid (lanes 10-11). Mice were sacrificed 18 hours (lanes 4-5), or 36 hours after beginning of treatment (lanes 3, 6-7, 10-11): total RNAs were collected from the spleens (with a >75% invasion of leukemic blasts as assessed by Giemsa staining) and analyzed by RNAse protection assay to check for expression of TRAIL. Faf and GAPDH were used as controls, not affected by these treatments. (B) Calu-3 cells (a human, non-small cell lung cancer derived cell line) were treated with VPA (1mM) for the indicated times. RNA was extracted and analyzed by quantitative PCR (Perkin-Elmer), using primers specific for TRAIL. GAPDH was used to normalize.

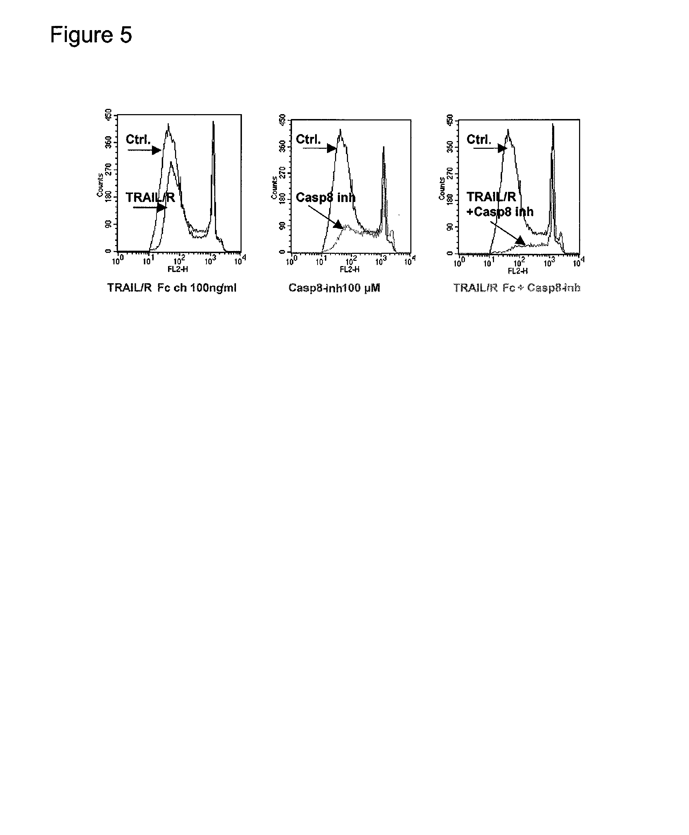

**Figure 5:** Leukemic blasts were obtained from spleens of secondary leukemic mice with a >75% invasion of tumor cells, as assessed by histological examination. Cells were put in culture and treated with VPA (1mM) for 48 hours, in the absence or in the presence of the indicated TRAIL and/or caspase 8 inhibitors as indicated. At the end of the treatment, apoptosis was measured as described above.

### RESULTS

Efficacy of HDAC inhibitor treatment was tested in a mouse leukemia model. Survival curves of mice with overt secondary leukemia showed that VPA treatment extended survival of leukemic mice in a statistically significant way (P < 0.01, Figure 1A). Additionally, VPA caused a dramatic reduction in the splenomegaly caused by blast infiltration (compare placebo with VPA-treated, Figure 1 B). To further analyze the activity of HDAC inhibitor treatment we used whole cell extracts prepared from the spleen (either normal spleen or leukemic spleen with >70% leukemic infiltration). VPA induces enhanced levels of histone acetylation both in normal mice, and in leukemic mice (Figure 2A). Furthermore, immuno-histochemistry analysis of VPA-treated leukemic mice using a monoclonal antibody against acetylated histones shows dramatic increase of histone acetylation in all cells upon VPA treatment, including leukemic blasts (Figure 2B). Cells obtained from leukemic spleens were then analyzed for the presence of apoptotic cells after treatment with or without VPA (TUNEL assay, Figure 3A, right panels). The results show that VPA induces massive apoptosis within leukemic spleens. To confirm these data, splenocytes from healthy mice (taken as control, Figure 3B, left panel), or from leukemic mice (>75% blasts, Figure 3B, right panel) were put in culture and then treated with VPA (1mM) for 48 hours. At the end of the treatment, apoptosis was measured by propidium iodide staining showing that VPA induces massive apoptosis of leukemic cells, but it is unable to induce apoptosis of normal splenocytes.

To understand the pathomechanism of selective induction of apoptosis in cancer cells, we investigated whether HDAC inhibitor treatment enhance TRAIL expression. As shown in figure 4A, whereas in vivo VPA treatment did not cause induction of TRAIL expression in the spleens of healthy mice (lanes 1-2), it induced TRAIL for the entire duration of treatment in two independent sets of leukemic mice analyzed (lanes 3-5, experiment 1; lanes 6-7, experiment 2) as strongly as upon 96 hours of retinoic acid treatment (lanes 8-9). Combined RA+VPA treatment did not further increase TRAIL expression (lanes 10-11). Furthermore, as shown in figure 4B, TRAIL is strongly induced in human CaLu-3 cancer cells by the treatment with VPA, demonstrating that TRAIL is also a target of VPA in non-leukemic cancer cells, and extending the principle that TRAIL induction as a consequence of VPA treatment may be broadly responsible for the induction of cell death in tumor cells. This was confirmed in experiments where leukemic cells could be rescued from apoptosis by inhibitors of TRAIL or caspase 8. As can be seen from the data of Figure 5, in experiments where the inhibition of TRAIL by a TRAIL receptor/Fc chimera (able to titrate out active TRAIL, and impairing signaling through the endogenous receptor), or by a caspase 8 inhibitor (directly blocking activation of caspase 8, downstream of activated TRAIL), was able to block partially (single treatments) or entirely (combined treatment) the effect of VPA, showing that the TRAIL pathway is a critical effector of VPA action.

### EXAMPLE 2

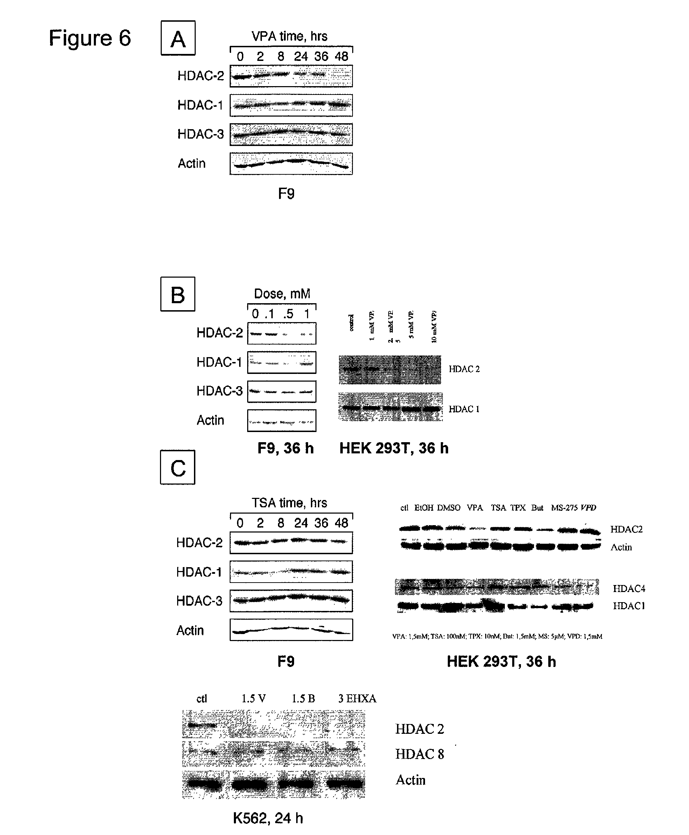

Valproic acid defines a class of HDAC inhibitors that induce proteosomal degradation of HDAC-2 (Figure 6-10).

The recruitment of histone acetyltranferases (HATs) and histone deacetylases (HDACs) is considered as a key element in the dynamic regulation of many genes playing important roles in cellular proliferation and differentiation. Hyperacetylation of the N-terminal tails of histones H3 and H4 correlates with gene activation whereas deacetylation can mediate transcriptional repression. Consequently, many diseases have been linked to changes in gene expression caused by mutations affecting transcription factors. Aberrant repression by leukemia fusion proteins such as PML-RAR, PLZF-RAR, AML-ETO and Stat5-RAR serves as a prototypical example in this regard, but it is becoming increasingly evident that similar events contribute to pathogenesis in many other types of cancer.

Mammalian histone deacetylases can be divided into three subclasses (Gray and Ekström, 2001). HDACs 1, 2, 3, and 8 which are homologues of the yeast RPD3 protein constitute class I. HDACs 4, 5, 6, 7, 9, and 10 are related to the yeast Hda 1 protein and form class II. Recently, several mammalian homologues of the yeast Sir2 protein have been identified forming a third class of deacetylases which are NAD dependent. All of these HDACs appear to exist in the cell as subunits of a plethora of multiprotein complexes. In particular, class I and II HDACs have been shown to interact with transcriptional corepressors mSin3, N-CoR and SMRT which serve as bridging factors required for the recruitment of HDACs to transcription factors.

The proteasome is widely recognised as the central enzyme complex of non-lysosomal protein degradation being an essential component of the ATP-dependent proteolytic pathway catalysing the rapid degradation of many rate-limiting enzymes, transcriptional regulators and critical regulatory proteins. It is essential for the rapid elimination of highly abnormal proteins, arising via mutation or by post-translational damage, and plays a primary role in the slower degradation of the bulk of proteins in mammalian cells. It is also critically involved in higher eukaryotes in antigen processing.

The present invention demonstrates the ability of certain HDAC inhibitors, in particular valproic acid, to selectively induce degradation of the HDAC-2 protein additionally to their HDAC inhibitory activity. This selective reduction in HDAC-2 protein levels is seen after stimulation with valproic acid and butyric acid but not with other HDAC inhibitors such as trichostatin A, trapoxin, and MS-275. Thus, HDAC inhibitors such as valproic acid inactivate HDAC-2 through two different mode of actions: they inhibit HDAC-2 activity and induce proteasomal degradation. The measurement of these combined activities may serve as a profiling tool for the identification of novel, and potentially more potent, inhibitors of enzymes having HDAC activity.

### METHODS

**Figure 6:** (A) F9 teratocarcinoma or HEK293T embryonic kidney cells were exposed for indicated times to 1 mM VPA. Protein levels of HDAC-2 and HDACs 1 or 3 for reference (HDAC8; N-CoR and Sin3 as data not shown) were determined by Westernblot analysis. (B) The dependency of reduction of HDAC-2 protein levels on the VPA dose was determined in F9 or HEK293T cells after 36 h of exposure. (C) The effect of other HDAC inhibitors on the amount of HDAC-2 protein was determined by using TSA (100 nM) in a time course analysis in F9 cells (left panel). The HDAC inhibitors TSA (100 nM), trapoxin (TPX, 10 nM), butyrate (1.5 mM) or MS275 (5 µM) were tested at a single time of exposure in HEK293T cells (right panel).

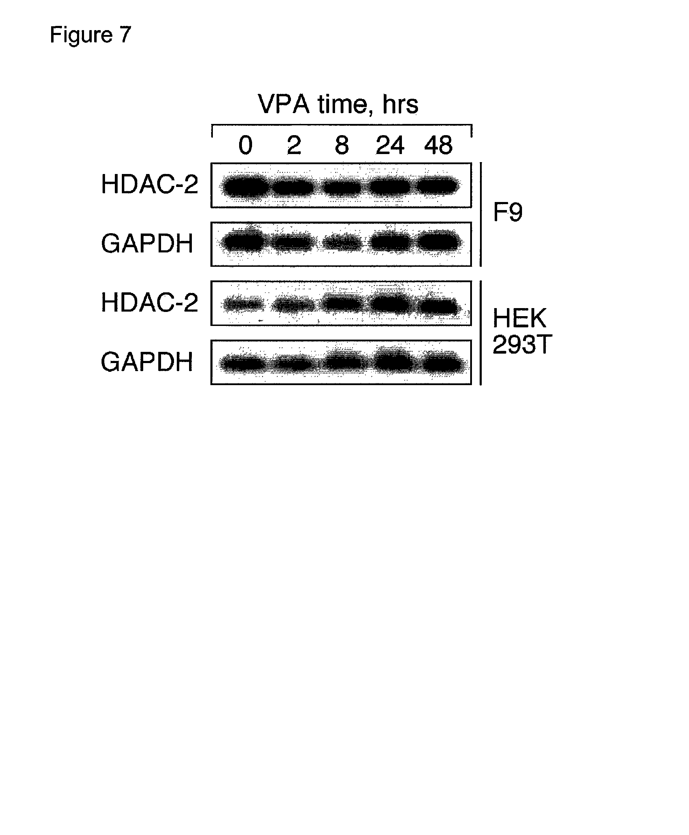

**Figure 7:** F9 cells or HEK 293T cells were treated for indicated times with 1 mM VPA and the levels of HDAC-2 mRNA were determined by Northern blot analysis from 5 µg polyA⁺ RNA.

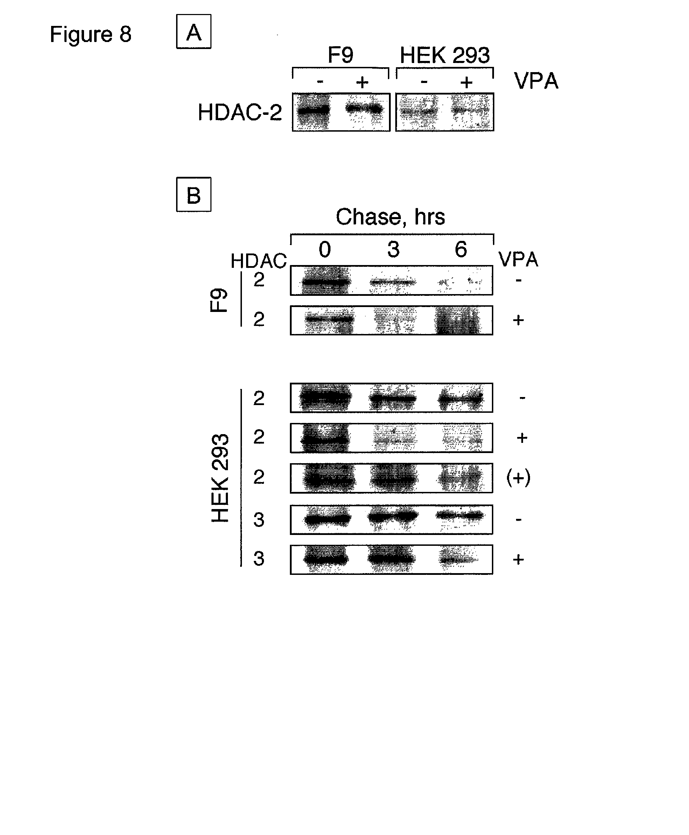

**Figure 8:** Synthesis and degradation rate of HDAC-2 were analyzed by pulse metabolic labeling with ³⁵S-methionine and pulse-chase analyses. ³⁵S-labelled HDAC-2 was detected by HDAC-2 specific immune precipitation followed by SDS-PAGE separation and autoradiography. (A) Pulse labeling for 1 h was performed in F9 and HEK293T cells which had been left untreated or were pretreated with 1 mM VPA for 24 h. (B) For pulse chase analysis cells were left untreated or pretreated for 24 h with 1 mM VPA and labeled with ³⁵S-methionine for an additional hour in the absence or presence of VPA. After removal of ³⁵S-methionine and addition of non-labeled methionine the elimination of radiolabeled HDAC-2 was followed over a period of 6 h. A pulse chase analysis was also performed in HEK293T cells without VPA pretreatment and addition of VPA (indicated by the (+) row) only at the time when the chase period was started. From HEK293T cell extracts also HDAC-3 was precipitated for reference.

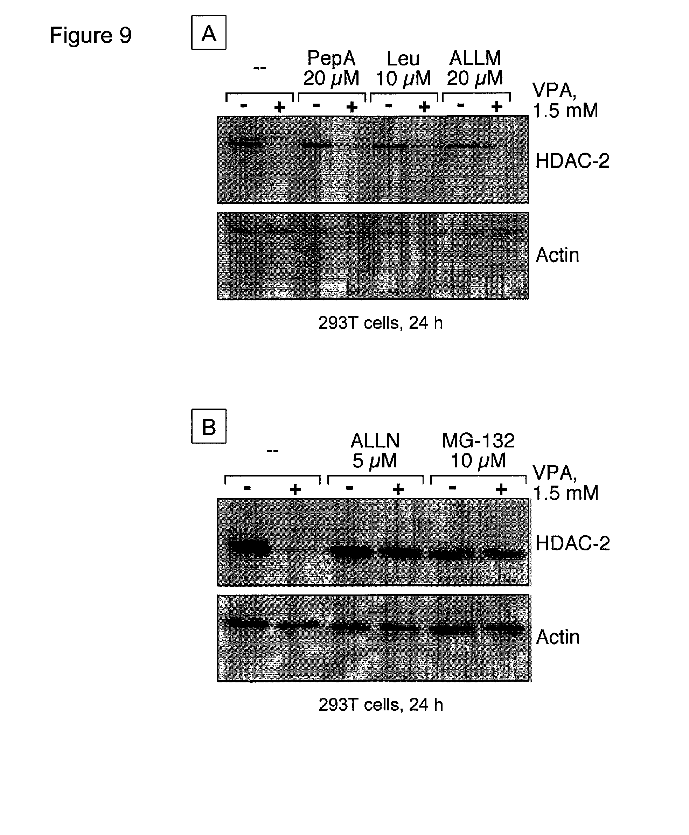

**Figure 9:** (A) HEK293T cells were treated for 24 h with VPA or left untreated. The cell penetrating protease inhibitors pepstatin A, leupeptin, or ALLM were added as indicated at the time of VPA addition. (B) Proteasome inhibitors ALLN or MG-132 were added at the indicated concentrations 4 h before cells were harvested. Protein levels of HDAC-2 were determined by Westernblot analysis.

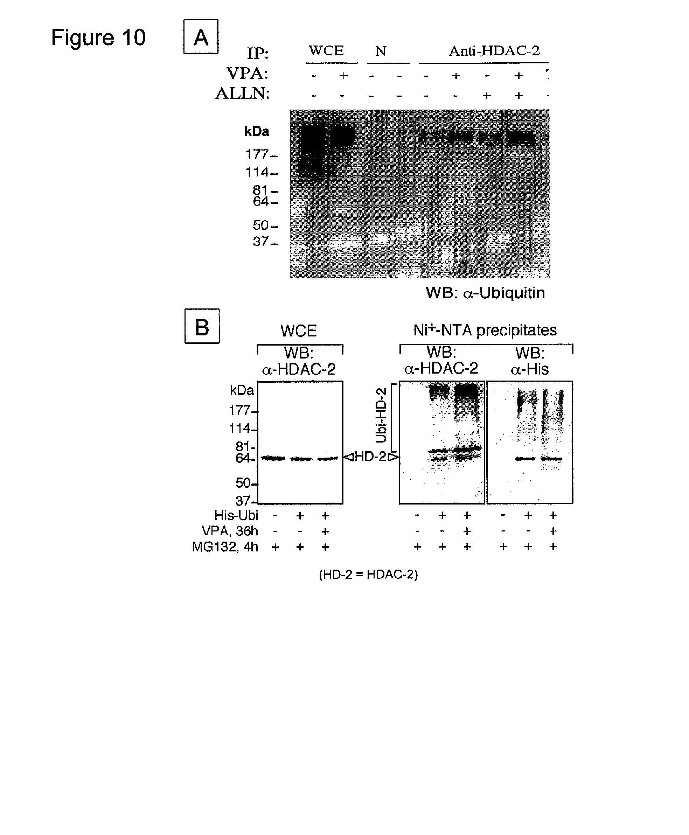

**Figure 10:** (A) The total amount of high molecular weight poly-ubiquitinated protein was not altered by VPA treatment of cells (left two lanes). The presence of ubiquitinated proteins in anti HDAC-2 immune precipitates is shown in the right part of the panel. Cells had either been left untreated, were exposed to VPA (1.5 mM, 24 h) or the proteasome inhibitor ALLN (5 µM, 24 h) or both. Ubiquitinated proteins in the precipitates were detected by Western blot analysis. For lane N a non-immune serum was used instead of the anti HDAC-2 antibody. (B) The presence of mono- and/ or oligo-ubiquitinated HDAC-2 upon VPA treatment of HEK293T cells was determined by ectopic expression of His₆-tagged ubiquitin followed by VPA-treatment for 36 h as indicated. The proteasome inhibitor MG-132 was added 4 h prior to analysis to enhance the accumulation of ubiquitinated proteins. Electrophoretic mobility and unaltered presence of non-ubiquitinated HDAC-2 were determined by Westernblot analysis of whole cell extracts (left panel). Ubiquitinated proteins were precipitated with Ni²⁺-NTA-agarose and analyzed for presence of ubiquitinated HDAC-2 (middle panel) by Westernblot analysis of precipitates. Anti-HDAC-2 immuno-reactive bands with the expected mobility of mono- or poly-ubiquitinated HDAC-2 are indicated by a bracket. Westernblots of precipitates were also probed with an antibody directed against the poly-histidine tag (right panel) to assure comparable expression of tagged ubiquitin and equal efficiency of incorporation of tagged ubiquitin into the general cellular pool of ubiquitinated proteins in control and VPA-treated cells.

### RESULTS

### Reduction of HDAC-2 protein levels by VPA treatment of cells and mice

In the course of testing whether VPA or other HDAC inhibitors would also, in addition to inhibiting HDAC enzyme activities, affect expression or HDAC protein levels e.g. in a compensatory feed-back loop, we found a consistent down-regulation of HDAC-2 protein levels in VPA treated cells. Reduction of protein levels to about 30 % of untreated cells is found after 24 h of VPA exposure and persists at least for up to 48 h (Figure 6A). HDAC-2 protein levels are reduced in murine F9 teratocarcinoma, human embryonic kidney HEK293T cells (Figure 6A), human K562 leukemia cells and mouse NIH3T3 cells. Protein levels of HDAC-1, HDAC-3, are not reduced but show, in some experiments, a transient induction (Figure 6A). HDAC-2 protein levels show a delayed response to VPA exposure suggesting the need for intermediary steps, e.g. the induction of intermediary gene products. VPA doses required for reduction of HDAC-2 protein levels are similar to those required for inhibition of HDAC enzyme activity, e.g. clear effects are detected at 0.5 to 1 mM. In some cells such as HEK 293T that tolerate higher doses of VPA a slightly more pronounced reduction is found if VPA concentrations are increased up to 5 or 10 mM (Figure 6B).

Reduction of HDAC-2 protein levels is specific for carboxylic acid HDAC inhibitors such as VPA, Butyrate and EXHA. Treatment with the hydroxamic acid TSA for up to 48 h does not reduce HDAC-2 levels in either F9 or HEK293T cells (Figure 6C). In F9 cells 48 h TSA is toxic as documented by cell loss and reduction in the actin loading control. The VPA-derived amide valpromide which does not inhibit HDACs does not affect HDAC-2 protein levels. Neither the cyclic tetrapeptide HDAC inhibitor trapoxin nor the anilide-based HDAC inhibitor MS-275 induces reduction of HDAC-2 protein levels.

However, under conditions that lead to reduction of HDAC-2 protein levels no response of HDAC-2 mRNA levels was found neither in F9 nor in HEK293T cells (Figure 7).

### Induced proteosomal degradation of HDAC-2 protein

Altered protein levels in the absence of alterations in steady state mRNA levels strongly suggest an effect on the rate of protein synthesis or protein degradation. HDAC-2 protein synthesis rate with and without VPA pretreatment (24 h) was compared by pulse labeling with ³⁵S-methionine in F9 or HEK293T cells. No substantial difference in HDAC-2 synthesis rate between control or VPA treated cells was found (Figure 8A).

Protein half-life times were determined by a pulse-chase analysis, both, in F9 and HEK293T cells. The half-life of HDAC-2 was substantially decreased by pretreatment of cells with VPA (Figure 8B). VPA had no effect on HDAC-2 protein degradation rate when added only at the time of chase (panel marked by (+) in Figure 8B) providing a further indication that the response of HDAC-2 protein levels to VPA is indirect rather than being a direct response, e.g. to a conformational change of HDAC-2. Degradation of HDAC-3 was not affected by VPA pretreatment of HEK293T cells (Figure 8B) consistent with a lack of reduction in steady state HDAC-3 protein levels upon VPA treatment.

To discriminate whether HDAC-2 degradation could be due to either protease-dependent or proteosomal degradation several inhibitors of proteases (Figure 9A) or the proteasome (Figure 9B) were applied in combination with VPA. Neither of the protease inhibitors pepstatin A, leupeptin or ALLM had an effect on control or VPA-repressed HDAC-2 protein levels (Figure 9A). Co-treatment of HEK293T cells with the proteasome inhibitors ALLN or MG-132 abolished VPA-induces degradation of HDAC-2 completely (Figure 9B). Thus, increased proteasomal degradation by an indirect mechanism involving synthesis of intermediary factors appears to be the most likely mechanism of VPA induced degradation of HDAC-2 protein.

A prerequisite of proteosomal degradation is the 'tagging' of the substrate protein for recognition by the proteasome. The most common mechanism of proteosomal targeting depends on poly-ubiquitination and, therefore, presence and VPA-dependent induction of ubiquitinated HDAC-2 was tested. First, immune precipitates prepared with an antibody directed against HDAC-2 were analyzed by Westernblot against ubiquitin for the presence of high molecular weight ubiquitinated proteins. Untreated cells contain a small amount of ubiquitin containing proteins that precipitate with an antibody against HDAC-2 (Figure 10A). Pretreatment of cells with either the proteasome inhibitor ALLN to prevent degradation of ubiquitinated proteins or with VPA increase the amount of this anti-ubiquitin reactive protein. Cotreatment with VPA and ALLN even further increases the amount of high molecular weight ubiquitinated proteins. The overall amount of cellular ubiquitinated proteins is, in contrast, moderately reduced by VPA treatment. TSA has no effect on the amount of immune precipitated anti-ubiquitin-reactive material. These data suggest that VPA-treatment induces ubiquitination of (a) protein(s) that precipitate with an antibody against HDAC-2.

The latter type of assay aiming at detecting the ubiquitinated forms of HDAC-2 was further improved by transient expression of His₆-tagged ubiquitin and using Ni²⁺-NTA agarose for precipitation (Figure 10B). Expression of recombinant ubiquitin does not substantially affect the total cellular amounts of HDAC-2 and, if present at all, ubiquitinated forms of HDAC-2 are below the detection limits of Westernblot analysis from whole cell extracts (left panel). Ni²⁺-NTA-agarose precipitates contain ubiquitinated proteins over almost the whole range of resolved protein sizes when Westernblots are probed with an anti His-tag antibody (right panel). Detection of ubiquitinated proteins is specific since no signal is seen if cells are not transfected to express the tagged ubiquitin. Gross abundance of ubiquitinated proteins is not altered by VPA treatment of cells. The prominent band at about 60 kDa most likely represents the ubiquitin concatamer encoded by the expression vector. If probed for presence of HDAC-2 the Ni²⁺-NTA-agarose precipitates clearly show the presence of oligo- and polyubiquitinated HDAC-2 (middle panel). The prominent band migrating between 64 and 81 kDa most likely represents mono-ubiquitinated HDAC-2 and a ladder of bands consistent with the expected mobility of any degree of HDAC-2 ubiquitination up to high molecular weight polyubiquitination is found. These bands are visible at low abundance in precipitates from untreated cells and are much more abundant if cells are pretreated with VPA. The detection is specific for ubiquitinated HDAC-2 since no such bands are visible if the cells do not express His-tagged ubiquitin. HDAC-2 specificity is established by the use of the proper antibody. The band comigrating with HDAC-2 just below 64 kDa most likely corresponds to unmodified HDAC-2 which could well be coprecipitated in larger corepressor complexes that contain several HDAC-2 molecules of which not all would have to be ubiquitinated. In summary, this part of the data shows that HDAC-2 can be ubiquitinated and degraded by the proteasome by a mechanism inducible by VPA but not by TSA.

### EXAMPLE 3

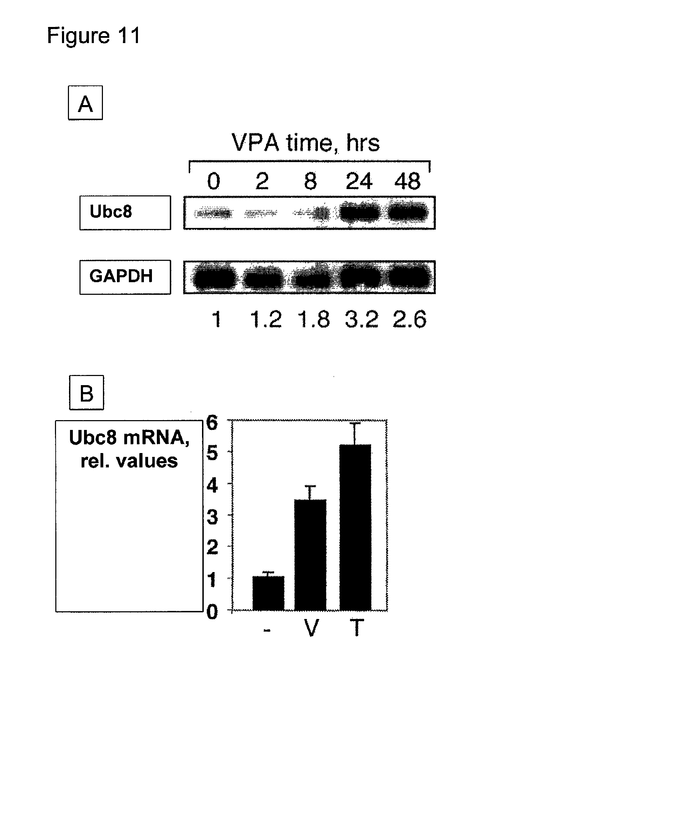

Valproic acid defines a class of HDAC inhibitors that induce expression of the E2 ubiquitin conjugating enzyme Ubc8, leading to ubiquitination of HDAC's 1-3 by the E3 ubiquitin ligase, RLIM (Figure 11-13).

Degradation of a protein via the ubiquitin pathway involves two successive steps: (1) tagging of the substrate by covalent attachment of multiple ubiquitin molecules, and (2) degradation of the tagged protein by the 26S proteasome complex with release of free and reusable ubiquitin.

The 26S proteasome is the key enzyme complex of the ubiquitin/ATP-dependent pathway of protein degradation. The 26S complex binds ATP and is responsible for the degradation of proteins that have been targeted for degradation by conjugation with ubiquitin. Ubiquitin is attached to a target protein by an isopeptide bond formed between the epsilon-amino group of lysine on the target and the C-terminal glycine residue of ubiquitin by a series of ubiquitin conjugating enzymes, E1, E2 and E3. Ubiquitin conjugating enzymes act in series by transferring a ubiquitin chain from one enzyme to the next, followed by the transfer of the activated ubiquitin chain from the E2 enzyme to the target protein. The mono-ubiquitinylated protein is then acted upon again and the same enzymes attach an additional ubiquitin molecule to the previous one. Ubiquitin conjugation continues resulting in a high molecular weight protein complex. This poly-ubiquitinylated product then becomes the target for rapid degradation by the 26S proteasome with concomitant recycling of ubiquitin catalysed by the isopeptidases (Ciechanover and Schwartz, 2002, Hepatology 35: 3-6).

The present invention demonstrates the ability of certain HDAC inhibitors, namely valproic acid, to selectively induce expression of the E2 ubiquitin conjugating enzyme Ubc8, without changing expression of the E3 ubiquitin ligase, RLIM. This induction of Ubc8 enhances ubiquitination of RLIM associated proteins, such as HDAC-2. On the other hand, some HDAC inhibitors (e. g. TSA) induce an enhanced proteosomal degradation of the E3 ligase RLIM, which consequently becomes the limiting factor for HDAC-2 turnover. These observations provide a plausible explanation for the differential effects on HDAC-2 protein levels observed upon treatment with different HDAC inhibitors.

### METHODS

**Figure 11 (A)** F9 cells were treated for indicated times with 1 mM VPA and abundance of Ubc8 mRNA was determined by Northern blot analysis from 5 µg polyA⁺ mRNA with a probe drived from the 3'-UTR of the cDNA. GAPDH mRNA was determined as reference. Phosphorimager analysis was performed for quantitative evaluation and relative values normalized for GAPDH are presented below the graph. One of two experiments with similar results is shown. (B) Ubc8 mRNA was also determined by real time PCR assuming an 1.5-fold amplification per cycle. The amplicon was part of the coding sequence of the Ubc8 mRNA. F9 cells were treated for 17 h with 1 mM VPA (V) or 100 nM TSA (T). Results were normalized for GAPDH expression. Average values ± range of duplicate determinations are shown.

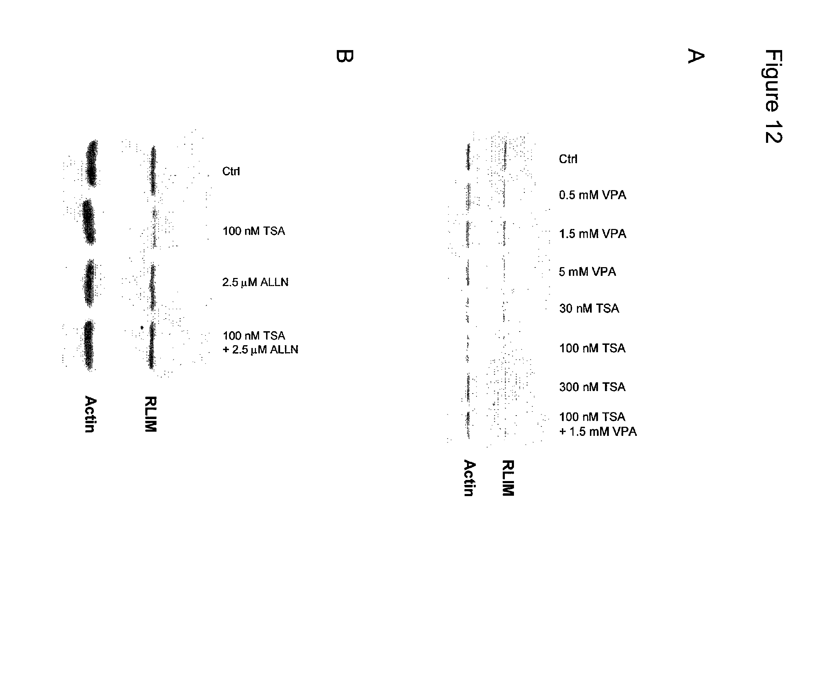

**Figure 12 (A)** RLIM protein levels were determined by Westernblot analysis after treatment of HEK293T cells for 24 hours with the HDAC inhibitors VPA (0.5, 1.5, or 5 mM), TSA (30, 100 or 300 nM) or a combination of VPA (1.5 mM) and TSA (100 nM). Actin protein levels are shown as a control for equal loading. (B) RLIM protein levels were determined by Westernblot analysis after treatment of HEK293T cells for 24 hours with the HDAC inhibitor TSA (100 nM), the proteasome inhibitor ALLN (2.5µM) or a combination of both. Actin protein levels are shown as a control for equal loading.

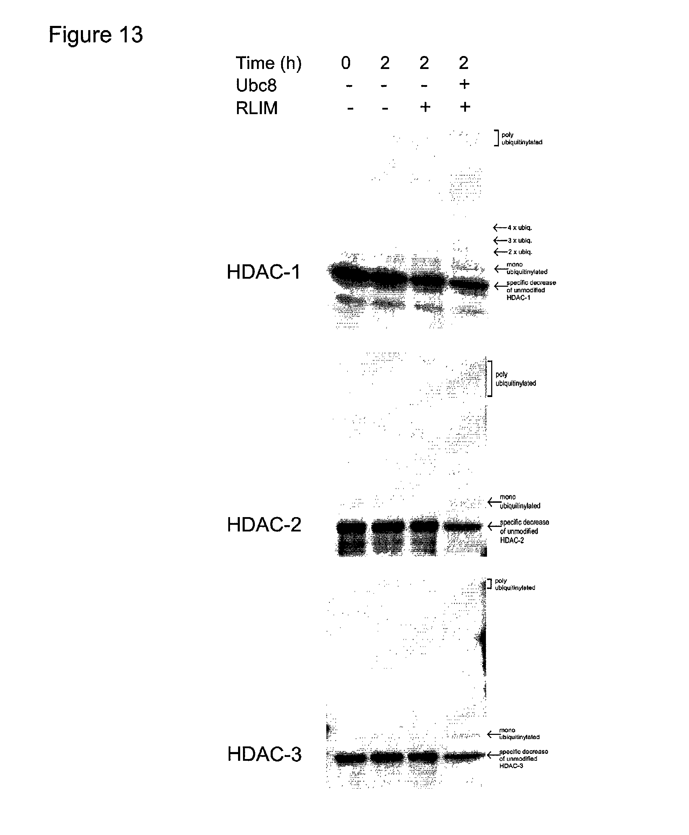

**Figure 13:** In vitro translated ³⁵S-methionine labeled HDACs 1-3 were incubated for 2 h in buffer with E1 ubiquitin ligase and ubiquitin only, or in reactions containing the recombinantly expressed E2 ubiquitin ligase Ubc8 without or together with the E3 ligase RLIM. For control (left lanes) reactions were stopped immediately after addition of ubiquitin and E1 ligase. Loss of input HDAC and appearance of high molecular weight radioactively labeled protein were determined by SDS PAGE followed by autoradiography.

### RESULTS

### Identification of ubiquitin ligases for HDAC's 1-3

Proteins of the ubiquitination machinery that can mediate HDAC-2 degradation were identified by two approaches. A systematic search for VPA inducible genes in F9 cells had revealed that expression of the gene encoding the E2 conjugating enzyme Ubc8 is inducible by VPA (Figure 11). Association of RLIM with the mSin3 corepressor (Ostendorff et al., 2002, Nature 416:99-103) had identified this E3 ubiquitin ligase as a prime candidate to be active also towards other components of corepressor complexes, e.g. HDACs. Interestingly, Ubc8 serves as E2 conjugating enzyme for RLIM ligase so that both proteins act synergistically.

In contrast to VPA, some HDAC inhibitors such as TSA induce an enhanced proteosomal degradation of the E3 ligase RLIM, which can be blocked with the 26S proteasome inhibitor ALLN (Fig. 12). Consequently, RLIM becomes the limiting factor for HDAC-2 turnover in the presence of TSA. Therefore, differential effects on HDAC-2 protein levels are observed upon treatment with different HDAC inhibitors.

In vitro ubiquitination assays with Ubc8 as E2 and RLIM as E3 ubiquitin ligases demonstrated that several class I HDACs, e.g. HDACs 1-3 (Figure 13), and the class II HDAC-4 are substrates for Ubc8/ RLIM. Both, the disappearance of the non-modified in vitro translated HDAC as well as the appearance of slower migrating proteins of the expected mobility of mono-, oligo-, and poly-ubiquitinated HDACs could be observed. Efficient ubiquitination was only found, if both recombinantly expressed proteins, Ubc8 and RLIM, were included in the reactions but not if only ubiquitin and an E1 ligase were present. In the case of HDAC-2 disappearance of unmodified protein and appearance of slowly migrating poly-ubiqitinated forms was most pronounced. Formation of ladders presumably representing various degrees of mono- and oligo-ubiquitination was observed better in the case of HDAC-1. In either case the data clearly show that Ubc8 and RLIM can ubiquitinate HDACs 1, 2, and 3 in cell free *in vitro* reactions.

### EXAMPLE 4

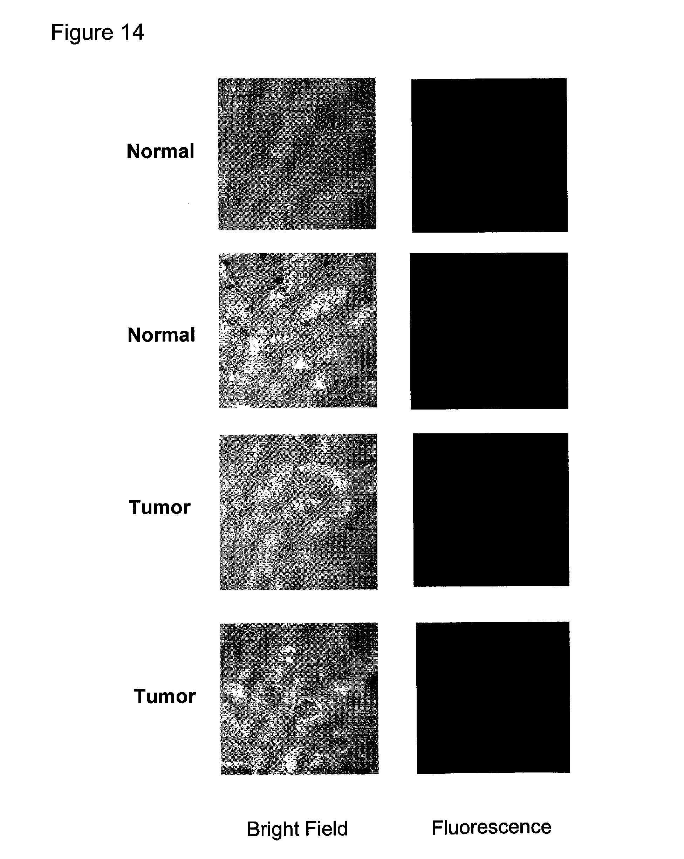

HDAC-2 is overexpressed in human colon cancer (Figure 14, Table 1)

Cancer of the colon is the second most frequently diagnosed malignancy in the United States as well as the second most common cause of cancer death. Surgery is the primary treatment and results in cure in approximately 50% of patients. Recurrence following surgery is a major problem and often is the ultimate cause of death. Elevated pretreatment serum levels of carcinoembryonic antigen (CEA) have a negative prognostic significance. Here we present evidence that HDAC-2 is overexpressed in over 80% of colon cancer probes investigated so far.

One aspect of the present invention is the use of HDAC-2 expression as a molecular marker to identify patients and tumor entities that may respond to a therapy with HDAC inhibitors. Furthermore, HDAC-2 expression in tumor cells can be used to monitor efficacy of HDAC inhibitor treatment in patients.

### METHODS

**Figure 14:** Paraffin colon cancer and matched normal tissue arrays (Biocat, Heidelberg) were deparaffinized with two changes of Xylene and a descending ethanol gradient (ethanol concentration: 100% 2x 5min.; 95% 3 min.; 70% 3min.) and were washed in PBS. Blocking was done in 10 % normal goat serum (DAKO Diagnostika, Hamburg) in PBS for 15 min. Incubation with 10µg/ml in 10%FCS/PBS of the HDAC-2-rabbit polyclonal antibody (Zymed, San Francisco) was done for one hour. After washing the slides 3x in PBS the sections were incubated with Cy3 coupled goat α-rabbit IgG F(ab')₂-Fragments (1:500 in 10% FCS/PBS) (Dianova, Hamburg) for 30 min. After the final washings in PBS (3x) the sections were covered with a coverslip. All photographs were taken with a Zeiss LSM 510 laserscanning inverted confocal microscope with the appropriate filters for Cy3.

### RESULTS

To investigate HDAC-2 expression in human tissue, biopsies from 46 colon cancer patients containing both tumor tissue and normal colon tissue from each patient were stained with an antibody against human HDAC-2 and a secondary antibody for immunofluorescence. As can be seen in Figure 14, very little staining for HDAC-2 expression could be detected in normal tissue, however over 80% of biopsies from tumor tissue displayed strong expression of HDAC-2 (Figure 14 and Table 1). This indicates that HDAC-2 over-expression is a very common feature in colon cancer and may be used as a prognostic and diagnostic marker in colon cancer patients. Furthermore, these results raise the hope that colon cancer patients benefit from a treatment that aims to reduce HDAC-2 expression in tumor tissue.

**TABLE 1:**

| Results from Immunofluorescence Detection of HDAC-2 Expression in Tumor Biopsies from Colon Cancer Patients (Compared to Matched Normal Tissue) | | | |
|---|---|---|---|
| | Normal HDAC-2 expression | HDAC-2 overexpressed | total |
| # samples | 8 | 38 | 46 |
| % samples | 17 | 83 | 100 |

## Claims

1. A method for the characterization of an HDAC inhibitor or a potential HDAC inhibitor comprising
determining in a sample the amount of a molecular marker
wherein the sample is derived from cells which have been treated with said HDAC inhibitor or potential HDAC inhibitor.

2. A method according to claim 1 wherein the molecular marker is selected from the group consisting of HDAC-2 RNA, HDAC-2 protein, Ubc8 RNA, UBC8 protein, RLIM RNA, RLIM protein, TRAIL RNA and TRAIL protein.

3. A method according to claim 1 or 2 wherein the sample is derived from tissue affected by a disorder.

4. A method according to claim 3 wherein the disorder is selected from but not restricted to the group consisting of skin cancer, melanoma, estrogen receptor-dependent and independent breast cancer, ovarian cancer, prostate cancer, renal cancer, colon and colorectal cancer, pancreatic cancer, head and neck cancer, small cell and non-small cell lung carcinoma, leukemias and other types of blood cell cancer and endocrine disease based on aberrant recruitment of histone deacetylase such as thyroid resistance syndrome.

5. A method according to anyone of claims 1 to 4 wherein the molecular marker is a ribonucleic acid and the amount of the molecular marker is determined by RT-PCR.

6. A method according to anyone of claims 1 to 4 wherein the molecular marker is a protein and the amount of the molecular marker is determined by use of an antibody directed against the molecular marker.

7. A method according to claim 6 wherein the amount of molecular marker is determined by Western Blotting, ELISA, immunohistochemistry and/or flow cytometry.

8. A method according to anyone of claims 1 to 7 further comprising the step of selecting the inhibitor if it has the activity of modulating the expression of the molecular marker.

9. A method according to anyone of claims 1 to 8 further comprising the step of determining in a reference sample the amount of said molecular marker wherein the reference sample is derived from cells which have not been treated with said HDAC inhibitor or potential HDAC inhibitor.

10. The use of a means for determining the amount of a molecular marker for profiling of HDAC inhibitors or potential HDAC inhibitors.

11. The use of a means for determining the amount of a molecular marker for diagnosing a disease.

12. The use of a means for determining the amount of a molecular marker for determining whether a treatment of a disorder with an HDAC inhibitor is to be started/continued or not.

13. The use of a means for determining the amount of a molecular marker for determining whether a treatment of a disorder with a therapy that targets a molecular marker is to be started/continued or not.

14. A diagnostic kit containing
(i) means for determining the amount of a molecular marker and
(ii) an HDAC inhibitor.
